# EUROPEAN PATENT APPLICATION

(11) **EP 4 230 203 A1**
(43) Date of publication of application: **23.08.2023**
(21) Application number: 21878806.5
(22) Date of filing: 14.10.2021
(51) Int. Cl.: A61K 31/4184, A61K 31/365, A61K 9/10, A61P 33/10

(54) **MICROSUSPENSION AGAINST PARASITES AND METHOD FOR OBTAINING SAME**

(30) Priority: 16.10.2020 BR 102020021181
(71) Applicant: Ipanema Industria de Produtos Veterinarios Ltda, 18190-000 Araçoiaba da Serra - SP (BR)
(72) Inventor: MASCARO, Claudio, 18190-000 Araçoiaba da Serra - SP (BR)
(74) Representative: Linage González, Rafael
(86) International application number: PCT/BR2021/050447
(87) International publication number: WO 2022/077087

(57) **Abstract**

The present invention refers to a microsuspension against parasites in animals and to a method for preparing same, more particularly intended for the veterinary industry. The microsuspension against parasites comprises: macrocyclic lactone and albendazole sulfoxide and has an average particle size of D₅₀ < 250 µm.

## Description

### APPLICATION FIELD

The present invention refers to a microsuspension against parasites in animals and its method of preparation, more particularly intended for the veterinary industry.

### STATE OF ART

The economic damage caused by worm infestation in animals is a worrying cause for livestock farmers. In this scenario, livestock farmers as well as governments are looking for effective solutions to minimize losses, as well as ensure correct handling of animals, avoiding their stress and ensuring the feasibility of good treatment.

Faced with the need to control parasites, numerous parasiticidal molecules were developed such as imidothiazoles, avermectins, benzoimidazoles, among others. Indeed, among the antiparasitics, one of the groups having the widest spectrum of action are the benzoimidazoles, as they are effective against nematodes, cestodes and trematodes.

Featured, benzoimidazoles have their structure characterized by being a bicycle composed of a benzene ring and an imidazole ring, as illustrated in Formula 1 below.

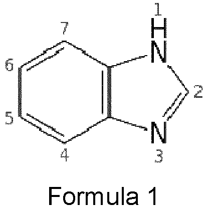

The presence of the sulfur atom in benzoimidazole derivatives has been widely studied, and they are important drugs capable of inhibiting larval stages in most nematodes. Among these derivatives is albendazole sulfoxide characterized by Formula 2, indicated below:

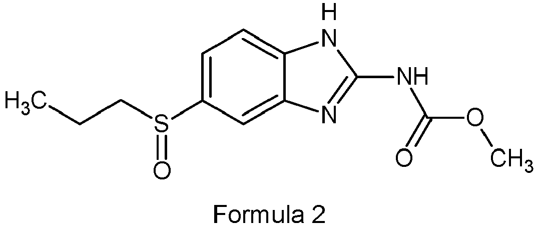

This compound had its synthetic description in patent US 3915986 and since then its application has been greatly explored as one of the long-spectrum anthelmintic compounds of low toxicity and high safety.

However, its low solubility in water due to its pka 7.8, generally limits commercial formulation to suspensions, pastes or granules for oral administration, or intraruminal administration to ruminants.

To overcome this limitation, however, an injectable solution of albendazole sulfoxide hydrochloride, which is the main active metabolite of albendazole, has been developed since mid-1979, as described in patent GB 2025223. Thus, the use of albendazole sulfoxide in the form of its hydrochloride solves a good part of the solubility problem. However, injectable solutions containing this salt cause wounds at the application site due to the high acidity of the required formulation, in addition to causing pain to the animal. Added to these facts, veterinary pharmaceutical formulations containing albendazole sulfoxide in the form of hydrochloride have stability problems, as it degrades over time, forming undesirable by-products, in addition to losing the potency of the active ingredient, especially if water is associated to the formulation (WU, 2009).

In patent application EP 0919231 injectable formulations of benzimidazoles are described using propylene glycol in presence of HCl.

Cyclodextrins also appear as an auxiliary to increase the solubility of ricobendazole, and these complexes and injectable formulations form the descriptions on the patent WO 2006022562.

Between the years 1980-1990, considerable advances in research for treatment of anthelmintics were conducted with macrocyclic lactones, especially Ivermectin, and its commercialization revolutionized the animal health industry. Ivermectin proved to be effective and safe against arthropod parasites at low dosage, but which currently encounters helminth resistance.

Thus, other avermectins and milbemycins emerged as a result of numerous researches, and also find applications in the animal health market. It remains to be concluded that macrolactones are widely used in more than 60 countries, including Brazil.

Nevertheless, doramectin (Formula 3) is a potent second-generation endectocide of the avermectin family and has been used for the treatment of endoparasite nematodes and ectoparasite insects, lice and ticks in cattle (GOUDIE, 1993). In commercial formulations it has been widely employed and used extensively in oral and pour-on forms.

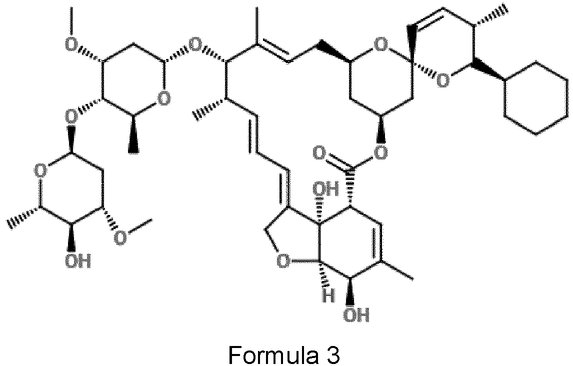

However, the resistance of helmintics to treatments and active principles (TAYLOR, 2002) has been a challenge to all animal breeders and the industries and researchers themselves.

To solve this problem, associations of pharmacologically active ingredients emerged, which in correct combination proved to be active and effective in the treatment of various classes of helminths.

Pertinently, there is a need to have a formulation capable of including macrolactones and albendazole sulfoxide.

Notably, combinations of macrocyclic lactones and benzimidazoles have been studied, in particular albendazole sulfoxide and avermectins. Patent BR 9406627 presents a composition of association of particulate albendazole sulfoxide or others, added to macrolactones, for oral administration, with casein and / or gelatin as dispersant.

One approach to overcoming the distinct chemical character of macrocyclic lactones and albendazole sulfoxide is to keep them separate in the formula, where one is suspended and the other soluble.

Patent BR 102013031277 represents a recent attempt to formulate the product combination ivermectin and albendazole sulfoxide. In such formulation, one active ingredient is dissolved in an organic liquid phase, while the other remains in suspension. This formulation comprises use of dimethylsulfoxide as solvent, N-methyl-2-pyrrolidone vehicle and ethyl alcohol as permeation agent. This formulation does not disclose the use of a dispersing agent, suspending agent or surfactant.

On the other hand, the association of doramectin and albendazole sulfoxide is little studied and consists of an association that combines the action of albendazole sulfoxide against the main gastrointestinal and pulmonary worms (nematodes) that affect cattle, in addition to Cestodes (tapeworms), *Cysticercus bovis* (embryo of *Taenia saginata), Moniezia expansa* and Trematodes (adult flukes). It presents action against the 3 stages of life cycle of parasites (adults, larvae - including hypobiotics and eggs). Cattle: Gastrointestinal nematodes: *Haemonchus* spp., *Trichostrongylus axei, Strongyloides* spp., *Cooperia* spp., *Oesophagostomum* spp., *Nematodirus* spp., *Chabertia* spp., *Bunostomum* spp., *Capillaria* spp.; Lung nematodes: *Dictyocalus* spp.; Cestodes: *Moniezia* spp.; Trematodes: *Fasciola hepatica; Cysticercos: Cysticercus bovis* (embryo of *Taenia saginata).* Doramectin targets the following parasites: Gastrointestinal nematodes (adults and L4 larvae): *Ostertagia ostertagi* (including inhibited larvae), *Haemonchus placei, Trichostrongylus axei, T. colubriformis, Cooperia oncophora, C. pectinata, C. punctata, C. surnabada (syn. mcmasteri), Nematodirus spathiger, Bunostomum phlebotomum, Strongyloides papillosus, Oesophagostomum radiatum, Trichuris spp.;* Lung nematodes (adults and L4 larvae): *Dictyocaulus viviparus.* Eye nematodes (adults): *Thelazia spp.;* Muscid larvae: (parasitic stages): *Hypoderma bovis, H. lineatum;* Sucking lice: *Haematopinus eurysternus, Linognathus vituli, Solenopotes capillatus.* Scabies mites: *Psoroptes bovis, Sarcoptes scabiei.* The veterinary medicinal product can also be administered as an aid for control of *Nematodirus helvetianus,* biting lice *(Damalinia bovis),* ticks *[Rhipicephalus (Boophilus) microplus]* and mange mites *Chorioptes bovis, Dermatobia hominis* and larvae of *Cochliomyia hominivorax.*

Thus, the association of these two substances can be indicated as a parasiticide for treatment of cattle, sheep and goats.

### OBJECTIVES OF INVENTION

In this sense, the objective was to develop an invention that refers to a composition of a macrocyclic lactone and albendazole sulfoxide constituting an effective injectable veterinary dosage form, involving only a few excipients, but having good resuspension, good viscosity, good syringeability, excellent stability and useful life, thus allowing greater anti-parasitic efficacy, also being commercially viable.

### SUMMARY

The present invention describes a simple and applicable method for preparing a stable microsuspended veterinary pharmaceutical form comprising macrocyclic lactone and albendazole sulfoxide having an average particle size of D₅₀ ≤ 250 µm.

### DETAILED DESCRIPTION

This invention refers to a new veterinary product from association of avermectin and benzoimidazole, more specifically, between doramectin and albendazole sulfoxide to combat parasites that attack animals. This product is a stable injectable microsuspended pharmaceutical formulation.

The process of the present invention consists of preparing a specific organic solvent system at room temperature, dissolving in it the macrocyclic lactone, preferably doramectin, and then adding albendazole sulfoxide, stirring using rotation between 150 and 600 r.p.m..

Thus, in this inventive process, a pharmaceutically stable microsuspended formulation is obtained, maintaining the individual pharmacological actions of the macrocyclic lactone, particularly doramectin and albendazole sulfoxide.

Therefore, the present invention presents an easy and simple way to prepare the formulation, in which, as it is a stable microsuspension, it avoids precipitation of the active albendazole sulfoxide in form of undesirable granules that could alter the resuspendability, and consequently interfering with the average dose injected into the animal.

This formulation, because it is stable and microsuspended, also provides excellent syringeability, reflecting in shorter handling time, absence of syringe clogging and less stress for the animals to be treated.

It is well known to the person skilled in the art that subcutaneous injections containing albendazole sulfoxide hydrochloride need to be at a low pH for its solubility, but due to the low pH, it causes pain in the animal, even promoting slow absorption and sustained plasmatic concentration since drug precipitation occurs in the injection site. In addition to this fact, injuries are observed at the injection site, which is quite harmful in view of problems associated with inflammation or possible infections.

The present invention overcomes this obstacle by presenting a pharmaceutical form with slow release, making use of benzimidazole, at neutral pH, avoiding such problems described above. Furthermore, the present invention guarantees the chemical stability of doramectin, since it is known that macrocyclic lactones can undergo hydrolysis easily, especially at acidic pH. Thus, this invention also solves the problem of macrolactone loss of potency over its shelf life.

It is also known that due to the low solubility of albendazole sulfoxide, the use of high polarity organic solvents, such as dimethylsulfoxide and N-methyl-2-pyrrolidone, having dielectric constants (ε) 48.9 and 32.0, respectively, is adopted as a technology. It is also known that the solubility coefficient is increased with increasing temperature. However, the use of this physical-chemical strategy solves the problem of apparent solubility, but causes deleterious and irreversible effects on the molecular structure of albendazole sulfoxide, causing its gradual decomposition.

Based on these chemical and pharmacological concepts, the present invention solves the problem of incompatibility and chemical degradation of the active principles (albendazole sulfoxide and doramectin) through the choice and correct association of excipients, in addition to providing a pharmaceutical form having safe, effective and prolonged action. The formulation is physically and chemically stable, excluding water, having a tissue-compatible pH, allowing two actives to coexist with stability in different phases.

The parasiticidal microsuspension according to the invention comprises, by mass, relative to the total mass of the composition, the components below:
- 0.5 to 10.0% macrocyclic lactone;
- 5.0 to 15.0% albendazole sulfoxide;
- 50.0 to 80.0% solvent;
- 15.0 to 45% of viscosity agent;
- 5.0 to 45.0% suspension stabilizing agent;
- preservatives.

The macrocyclic lactones according to the invention can be chosen from the group comprising doramectin, moxidectin, ivermectin, abamectin, eprinomectin and others, or mixtures thereof. Preferably, doramectin is used.

The solvents for preparing the formulation of this invention can be chosen from the group of hexylene glycol, acetone, isopropanol, methyl ester, N-dimethylacetamide, acetonitrile, dipropylene glycol monomethyl ether, butyrolactone, glycerolformal, benzyl benzoate, N-methyl-2-pyrrolidone, triacetin. Preferably, glycerolformal and / or methyl ester are used.

The viscosity agents can be chosen from glycerin, gelatin, collagen, propylene glycol, ammonium lactate, butylene glycol and D-panthenol. Preferably, glycerine and / or propylene glycol are used.

The suspension stabilizing agents used in this invention can be selected from vinylpyrrolidone, sorbitol, carbopol, hydroxyethyl cellulose, vinyl copolymers, macrogol derivatives, polyethylene glycol derivatives. Preferably polyethylene glycol derivatives are used, more preferably PEG-400 and / or PEG-200 and / or sorbitol.

The preservatives contained in the formulation can be chosen from the paraben class, particularly propyl and / or methyl p-hydroxybenzoate.

The present invention describes a simple and applicable method for preparing a stable microsuspension veterinary pharmaceutical form, where the macrocyclic lactone is in solution, the albendazole sulfoxide is in suspension having an average effective particle size of D50 ≤ 250 µm, preferably D50 ≤ 100 µm, more preferably D50 ≤ 50 µm, and even more preferably D50 ≤ 25 µm.

It is also part of the inventive scope that the prepared pharmaceutical form presents a viscosity between 10-100 cP, preferably 20 to 50 cP.

The microsuspension formulation according to the invention comprises, by weight, relative to the total weight of the composition, the following components:
- 0.5 to 10.0% doramectin;
- 5.0 to 15.0% albendazole sulfoxide;
- 50.0 to 80.0% glycerolformal;
- 15.0 to 45.0% propylene glycol;
- 15 to 45% polyethylene glycol, preferably PEG-200;
- 0.005 to 0.015% propyl p-hydroxybenzoate;
- 0.05 to 0.015% methyl p-hydroxybenzoate.

According to an embodiment of the invention, the microsuspension formulation according to the invention comprises, by mass, relative to the total mass of the composition, the following components:
- 0.5 to 10.0% doramectin;
- 5.0 to 15.0% albendazole sulfoxide;
- 31.0 to 72.0% glycerolformal;
- 23 to 38% glycerin;
- 16 to 44% sorbitol; and
- preservatives.

According to another embodiment, the microsuspension according to the invention comprises, by mass, relative to the total mass of the composition, the following components:
- 0.5 to 10.0% doramectin;
- 5.0 to 15.0% albendazole sulfoxide;
- 50.0 to 80.0% glycerolformal;
- 15.0 to 45.0% propylene glycol;
- 5.0 to 15.0% polyethylene glycol, preferably PEG-400;
- 0.05 to 0.015% methyl p-hydroxybenzoate;
- 0.005 to 0.015% propyl p-hydroxybenzoate.

According to yet another embodiment, the microsuspension according to the invention comprises, by mass, relative to the total mass of the composition, the following components:
- 0.5 to 10.0% doramectin;
- 5.0 to 15.0% albendazole sulfoxide;
- 45.0 to 75.0% methyl ester;
- 13.0 to 42% propylene glycol;
- 5.0 to 15.0% polyethylene glycol, preferably PEG-400;
- 0.05 to 0.015% methyl p-hydroxybenzoate;
- 0.005 to 0.015% propyl p-hydroxybenzoate.

The preferred components are illustrated in Table 1 below (component, concentration variation and their function in the formulation), by mass, relative to the total mass of the mixture.

**TABLE 1**

| **Ingredient** | **Function** | **Percentage (% by mass)** |
|---|---|---|
| Doramectin | Active Ingredient | 0.5 - 10.0 |
| Albendazole Sulfoxide | Active Ingredient | 5.0 - 15.0 |
| Glycerolformal | Solvente | 50.0 - 80.0 |
| PEG | Suspension Stabilizer | 5.0 - 15.0 |
| Propylene Glycol | Viscosity Agent | 15.0 - 45.0 |
| Methyl and propyl hydroxybenzoate | Preservative | 0.05 - 0.015 |

It is considered stable a pharmaceutical formulation that is approved in stability tests under conditions at a temperature of 30 °C ± 2 °C at 65% RH ± 5% (according to Climatic Zone IV) in a period equivalent to 24 months. In accelerated tests, 40 °C ± 2 °C at 75% RH ± 5% for 6 months, or ultra-accelerated at 50 °C ± 2 °C at 90% RH ± 5% for 03 months, can be used.

According to the invention, it was decided to carry out the stability test using 50 °C ± 2 °C at 90% RH ± 5% for 03 months, and the results are illustrated in Table 2 below (stability data of Formulations 01-03 under conditions 50 °C ± 2 °C at 90% RH ± 5%), demonstrating that the actives in the formulation remained stable within the limits required for approval of the stability period.

**TABLE 2**

| | **Initial** | | **1st month** | | **2nd month** | | **3rd month** | |
|---|---|---|---|---|---|---|---|---|
| Batch | ABZSO | Dora | ABZSO | Dora | ABZSO | Dora | ABZSO | Dora |
| **01** | 8.65 | 0.920 | 8.52 | 0.908 | 8.38 | 0.896 | 8.33 | 0.895 |
| **02** | 8.63 | 0.920 | 8.53 | 0.908 | 8.38 | 0.898 | 8.32 | 0.896 |
| **03** | 8.63 | 0.920 | 8.52 | 0.912 | 8.37 | 0.901 | 8.32 | 0.899 |

Observations:
(1) the unit of measurement is (m/m) (%);
(2) ABZSO is an abbreviation for the name albendazole sulfoxide;
(3) Dora is an abbreviation for the name doramectin.

Another aspect that concerns this invention is the acceptability of the viscosity of the formulation. Obeying the preparation of the pharmaceutical formulation, it will present excellent syringeability, which is the ability of a product to be successfully administered by means of a suitable syringe and needle.

As a consequence, optimal syringeability is dependent on the viscosity of the solution and the size of the particles, which are usually measured by laser diffraction, and are measured in micrometers or nanometers. D₅₀ is the mean particle diameter.

In a specific objective, the prepared formulation, administered subcutaneously, is intended for treatment and prevention of parasites caused by endoparasites and ectoparasites.

In particular, in this invention the average size of the particles must be less than 100 micrometers, ranging from 1 to 50 micrometers.

### METHOD OF PREPARING A PHARMACEUTICAL FORM

The preparation process of the present invention consists of preparing a specific organic solvent system at room temperature, dissolving the doramectin in it and then adding the albendazole sulfoxide, under constant stirring.

In the present invention, the preparation of the pharmaceutical formulation is simple and easy to perform. To obtain said formulation, it is necessary to subject albendazole sulfoxide to micronization before preparing the formulation.

Such steps for preparation of the injectable pharmaceutical formulation according to this invention occur at room temperature and are described below:
a) under agitation, one or more solvents, the viscosity agent and the suspension stabilizer are mixed in a suitable reactor;
b) after filtering the solution obtained in the previous step, the preservatives are dissolved under stirring until complete dissolution;
c) the macrocyclic lactone is then dissolved in the liquid mixture obtained in step (c) under stirring until complete dissolution, followed by filtration;
d) under constant agitation, micronized albendazole sulfoxide having average particle size D₅₀ ≤ 250, preferably D50 ≤ 50 µm, maintaining constant agitation until the final suspension is obtained.

According to a preferred embodiment of the method for obtaining the microsuspension according to the invention, it has the following steps:
a) under agitation from 150 to 600 r.p.m. the solvent, the viscosity agent and the suspension stabilizer are mixed in a suitable reactor;
b) after filtering the solution obtained in the previous step, the preservatives are dissolved under stirring until complete dissolution;
c) doramectin is then dissolved in the liquid mixture obtained in step (c) under stirring until complete dissolution, followed by filtration;
d) under constant agitation, micronized albendazole sulfoxide having average particle size D₅₀ ≤ 250 µm, preferably D50 ≤ 50 µm, is added, maintaining constant agitation until the final suspension is obtained.

The formulations were exhaustively tested, and during studies an appropriate formulation was found having excellent stability, ideal viscosity, and commercially desired conditions. Another important variable for good acceptance of the formula is to have good resuspendability, which surprisingly was achieved by using a suspension stabilizing agent. That is, the formulation could be easily resuspended with only moderate agitation, tested in 50 mL or 500 mL bottles that contained the formulation.

The formulations according to the invention serve for low injection volume, such as 1 mL for 50 kg of animal body weight.

The formulation according to the invention proved to be effective against *Ostertagia ostertagi, Cooperia pectinata, Cooperia punctata, Trichostrongylus axei, Trichostrongylus colubriformis, Oesophagostomum radiatum, Haemonchus placei, Dictyocaulus viviparus* and *Moniezia expansa;* and doramectin is effective against the endoparasites *Ostertagia ostertagi, Cooperia pectinata, Cooperia punctata, Trichostrongylus axei, Trichostrongylus colubriformis, Oesophagostomum radiatum, Haemonchus placei* and *Dictyocaulus viviparus,* and the ectoparasites *Dermatobia hominis, Cochliomyia hominivorax larvae* and *Rhipicephalus (Boophilus) microplus.*

Below are examples that serve to illustrate the scope of the invention, and should not be used for purposes of limiting the invention.

### EXAMPLE 1

### PREPARATION OF INJECTABLE FORMULATION

Initially, albendazole sulfoxide was micronized until an average particle size of particles D₅₀ = 3.3 µm was obtained.

### PHASEI

In a vase under agitation using rotation of 300 r.p.m., 73.2 kg of glycerolformal, 31.08 kg of propylene glycol and 11.30 kg of PEG-400 were added. Stirring was maintained at 300 r.p.m. for 30 minutes.

### PHASE II

80 kg obtained in Phase I were filtered through a filter = 0.2 µm, and 100 g of methyl p-hydroxybenzoate and 10 g of propyl p-hydroxybenzoate were added, being stirred until complete dissolution. Then 1.06 kg of doramectin was added and stirred at 300 r.p.m. until complete dissolution and filtration through a 0.2 µm filter.

### PHASE III

Under constant stirring at 300 r.p.m., 10.0 kg of sterile micronized albendazole sulfoxide having particle size D50 = 3.3 µm were loaded onto Phase II. Another 24.40 kg of Phase I solution was charged. It was stirred at 300 r.p.m. for 30 minutes, thus obtaining the final suspension.

### EXAMPLE 2

Initially, albendazole sulfoxide was micronized until an average particle size of particles D50 = 3.3 µm was obtained.

### PHASE I

In a vase under agitation using rotation of 300 r.p.m., 71.80 kg of glycerolformal, 23.5 kg of glycerin and 16.3 kg of sorbitol were added. Stirring was maintained at 300 r.p.m. for 30 minutes.

### PHASE II

80 kg obtained in Phase I were filtered through a 0.2 µm filter and 100 g of methyl p-hydroxybenzoate and 10 g of propyl p-hydroxybenzoate were added, being stirred until complete dissolution. Then 1.06 kg of doramectin was added and stirred at 300 r.p.m. until complete dissolution and filtration through a 0.2 µm filter.

### PHASE III

Under constant stirring at 300 r.p.m., 10.0 kg of sterile micronized albendazole sulfoxide having particle size D50 = 3.3 µm were loaded onto Phase II. An additional 24.47 kg of Phase I solution was charged. It was stirred at 300 r.p.m. for 30 minutes, thus obtaining the final suspension.

### EXAMPLE 3

### PHASE I

In a vase under agitation using rotation of 300 r.p.m., 50.20 kg of methyl ester, 23.5 kg of propylene glycol and 16.3 kg of sorbitol were added. Stirring was maintained at 300 r.p.m. for 30 minutes.

### PHASE II

80 kg obtained in Phase I were filtered through a 0.2 µm filter and 100 g of methyl p-hydroxybenzoate and 10 g of propyl p-hydroxybenzoate were added, being stirred until complete dissolution. Then 1.06 kg of doramectin was added and stirred at 300 r.p.m. until complete dissolution and filtering through a 0.2 µm filter.

### PHASE III

Under constant stirring at 300 r.p.m., 10.0 kg of sterile micronized albendazole sulfoxide having particle size D50 = 3.3 µm were loaded onto Phase II. An additional 24.47 kg of Phase I solution was charged. It was stirred at 300 r.p.m. for 30 minutes, thus obtaining the final suspension.

Syringeability tests were conducted with the formulation obtained in Example 1 in a 5 mL syringe using a needle (40 × 1.2 mm) or (40 × 1.6 mm). In Table 3 below, it is observed that none of the tests performed showed any clogging.

**TABLE 3**

| **Test** | **Formulation 01** | **Formulation 02** | **Formulation 03** |
|---|---|---|---|
| Viscosity (cP) | 41.00 | 40.00 | 38.93 |
| Average Particle Size (µm) | 2.58 | 2.57 | 2.57 |
| Appearance | White to slightly yellow suspension | White to slightly yellow suspension | White to slightly yellow suspension |
| Resuspension | Full Resuspension | Full Resuspension | Full Resuspension |
| Syringability | No clogging | No clogging | No clogging |

## Claims

1. Microsuspension against parasites, **characterized by** comprising macrocyclic lactone and albendazole sulfoxide having an average particle size D₅₀ ≤ 250 µm.

2. Microsuspension against parasites, according to claim 1, **characterized by** albendazole sulfoxide has an average particle size of D50 ≤ 50 µm.

3. Microsuspension against parasites, according to claim 1, **characterized by** it has a viscosity between 10 cP to 100 cP, preferably 20 cP to 50 cP.

4. Microsuspension against parasites, according to claim 1, **characterized by** the composition of the macrocyclic lactone can be chosen from the group comprising doramectin, moxidectin, ivermectin, abamectin, eprinomectin and others, or even mixtures thereof.

5. Microsuspension against parasites, according to claim 4, **characterized by** the microsuspension uses doramectin.

6. Microsuspension against parasites, according to claim 1, **characterized by** the composition further comprises solvents chosen from the group comprising hexylene glycol, acetone, isopropanol, methyl ester, N-dimethylacetamide, acetonitrile, glycerolformal, dipropylene glycol monomethyl ether, butyrolactone, benzyl benzoate, N-methyl-2-pyrrolidone, triacetin.

7. Microsuspension against parasites, according to claim 6, **characterized by** using glycerolformal and / or methyl ester.

8. Microsuspension against parasites, according to claim 1, **characterized by** further comprising viscosity agents that can be chosen from glycerin, propylene glycol, gelatin, collagen, ammonium lactate, butylene glycol, D-panthenol.

9. Microsuspension against parasites, according to claim 8, **characterized by** using glycerin and / or propylene glycol.

10. Microsuspension against parasites, according to claim 1, **characterized by** further comprising suspension stabilizing agents that can be selected from vinylpyrrolidone, sorbitol, carbopol, hydroxyethyl cellulose, vinyl copolymers, macrogol derivatives, polyethylene glycol derivatives.

11. Microsuspension against parasites, according to claim 10, **characterized by** using polyethylene glycol and / or sorbitol derivatives.

12. Microsuspension against parasites, according to claim 11, **characterized by** it comprises PEG-400, PEG-200, and / or sorbitol.

13. Microsuspension against parasites, according to any of the preceding claims, **characterized by** it comprises, by mass, relative to the total mass of the composition, the following components:
0.5 to 10.0% macrocyclic lactone;
5.0 to 15.0% albendazole sulfoxide;
50.0 to 80.0% solvent;
15.0 to 45.0% viscosity agent;
5.0 to 45.0% suspension stabilizing agent;
preservatives.

14. Microsuspension against parasites, according to any of the preceding claims, **characterized by** it comprises, by mass, relative to the total mass of the composition, the following components:
0.5 to 10.0% doramectin;
5.0 to 15.0% albendazole sulfoxide;
50.0 to 80.0% glycerolformal;
15.0 to 45.0% propylene glycol;
15.0 to 45.0% polyethylene glycol, PEG-200;
0.005 to 0.015% propyl p-hydroxybenzoate;
0.05 to 0.015% methyl p-hydroxybenzoate.

15. Microsuspension against parasites, according to any of the preceding claims, **characterized by** it comprises, by mass, relative to the total mass of the composition, the following components:
0.5 to 10.0% doramectin;
5.0 to 15.0% albendazole sulfoxide;
31.0 to 72.0% glycerolformal;
23 to 38% glycerin;
16 to 44% sorbitol; and
preservatives.

16. Microsuspension against parasites, according to any of the preceding claims, **characterized by** it comprises, by mass, relative to the total mass of the composition, the following components:
0.5 to 10.0% doramectin;
5.0 to 15.0% albendazole sulfoxide;
50.0 to 80.0% glycerolformal;
15.0 to 45.0% propylene glycol;
5.0 to 15.0% polyethylene glycol, PEG-400;
0.05 to 0.015% methyl p-hydroxybenzoate;
0.005 to 0.015% propyl p-hydroxybenzoate.

17. Microsuspension against parasites, according to claim 1, **characterized by** it comprises, by mass, in relation to the total mass of the composition, the following components:
0.5 to 10.0% doramectin;
5.0 to 15.0% albendazole sulfoxide;
45.0 to 75.0% methyl ester;
13.0 to 42% propylene glycol;
5.0 to 15.0% polyethylene glycol, PEG-400;
0.05 to 0.015% methyl p-hydroxybenzoate;
0.005 to 0.015% propyl p-hydroxybenzoate.

18. Method of obtaining microsuspension against parasites, described in claims 1 to 17, **characterized by** it occurs at room temperature and presents the steps described below:
a) under agitation, one or more solvents, the viscosity agent and the suspension stabilizer are mixed in a suitable reactor;
b) after filtering the solution obtained in the previous step, the preservatives are dissolved under stirring until complete dissolution;
c) the macrocyclic lactone is then dissolved in the liquid mixture obtained in step (c) under stirring until complete dissolution, followed by filtration;
d) under constant agitation, micronized albendazole sulfoxide having average particle size D₅₀ ≤ 250 µm, preferably D50 ≤ 50 µm, is added, maintaining constant agitation until the final suspension is obtained.

19. Method of obtaining microsuspension against parasites, according to claim 18, **characterized by** it occurs at room temperature and presents the steps described below:
a) under agitation from 150 to 600 r.p.m. the solvent, the viscosity agent and the suspension stabilizer are mixed in a suitable reactor;
b) after filtering the solution obtained in the previous step, the preservatives are dissolved under stirring until complete dissolution;
c) doramectin is then dissolved in the liquid mixture obtained in step (c) under stirring until complete dissolution, followed by filtration;
d) under constant agitation, micronized albendazole sulfoxide having average particle size D₅₀ ≤ 250 µm, preferably D50 ≤ 50 µm, is added, maintaining constant agitation until the final suspension is obtained.
